# EUROPEAN PATENT APPLICATION

(11) **EP 3 034 028 A1**
(43) Date of publication of application: **22.06.2016**
(21) Application number: 15191355.5
(22) Date of filing: 23.10.2015
(51) Int. Cl.: A61B 34/00

(54) **A MULTI-DEGREE OF FREEDOM SURGICAL INSTRUMENT FOR MINIMALLY INVASIVE SURGERY**

(30) Priority: 17.12.2014 CN 201420805476 U; 17.12.2014 CN 201410788732
(71) Applicant: Suzhou Kang Multi Robot Co Ltd, Suzhou City, Jiangsu (CN)
(72) Inventor: Du, Zhijiang, Suzhou Jiangsu (CN); Yan, Zhiyuan, Suzhou Jiangsu (CN); Wang, Jianguo, Suzhou Jiangsu (CN); Wang, Weidong, Suzhou Jiangsu (CN)
(74) Representative: Baldwin, Mark

(57) **Abstract**

A multi-DOF surgical instrument for minimally invasive surgery which comprising a hand held portion 10, a power portion 20 which is fix connected with the hand held portion 10, a thin linking rod 30 which is fix connected with the power portion 20, an operative end portion 40 which is connected with the thin linking rod 30, and an operative system for controlling the operation of the power portion 20. Wherein, the hand held portion and the power portion are wire connected with the operative system, the operation of the hand held portion triggers operations of encoders on the two joints provided on the instrument. The encoder collects the operation information of the joints and feeds back to the operative system , and the power portion receives signal from the operative system and drive the operative end portion to perform pitch, yaw, clamping motion.

## Description

### FIELD OF THE INVENTION

The present invention relates to surgical instruments, more particularly, relates to a multi-degree of freedom (DOF) surgical instrument for minimally invasive surgery.

### BACKGROUND OF THE INVENTION

A minimally-invasive procedure (MIP) typically involves use of arthroscopic (for joints and the spine) or laparoscopic devices and remote-controlled manipulation of instruments with indirect observation of the surgical field through an endoscope or large scale display panel, and is carried out through the skin or through a body cavity or anatomical opening. By use of a MIP, a patient may require only a band-aid on the incision, rather than multiple stitches or staples to close a large incision. This usually results in less infection, a quicker recovery time and shorter hospital stays.

Special medical equipment, such as fibre optic cables, miniature video cameras and special surgical instruments handled via tubes inserted into the body through small openings in its surface may be used to perform the surgery. However, the degree of freedom provided by existing surgical instruments is limited. These surgical instruments typically do not provide pitch and yaw movement. Therefore, they are clumsy and can seriously affect the quality and accuracy of the surgery. As such, an improvement in these surgical instruments is needed.

### SUMMARY OF THE INVENTION

An object of the invention is to provide a compact multi-degree of freedom (DOF) surgical instrument for minimally invasive surgery that is capable of yaw, pitch and clamping motions that increase the degree of freedom of movement available during surgery in order to increase the quality and efficiency of the surgery, or to at least provide an alternative to existing products on the market. In addition, turning of the arm of the surgeon can be reflected as a rotation motion.

The invention provides a multi-DOF surgical instrument for minimally invasive surgery which comprising a hand held portion, a power portion which is fix connected with the hand held portion, a thin linking rod which is fix connected with the power portion, an operative end portion which is connected with the thin linking rod, and an operative system (not shown) for controlling the operation of the power portion;
the hand held portion which comprising a first linking rod that one end of the linking rod is fix connected with the power portion, a second linking rod which through a first joint, is rotatably connected with the first linking rod, a third linking rod which through a second joint, is rotatably connected with the second linking rod, a clamping unit which through a clamping cap, is fixed mounted on the top of the third linking rod, and encoders which are fixed mounted on the first joint and the second joint;
the clamping unit which comprising a base panel, the base panel is provided with a pair of symmetrically opposing planar linkage mechanisms, each of the linkage mechanisms which comprising a mover lever and a lower lever, one end of the mover lever is rotatably mounted on the base panel, and the other end of the mover lever is hinged to one end of the lower lever, and the other end of the lower lever is rotatably mounted on a protruding end of a potentiometer, the potentiometer is fix mounted on the base panel, a spring is provided in between the mover levers of the pair linkage mechanisms. The two ends of the spring are abutting against the two mover levers, and the side wall of the mover lever is provided with a clamping piece; and
the hand held portion and the power portion are both wire connected with the operative system. The operation of the hand held portion drives the encoder on the joints to operative, the encoder collects the operative information of the joints and then feeds back to the operative system, after the power portion receives signals from the operative system, the power portion drives the operative end portion to complete a yaw, pitch and clamping motion.

Optionally, the power portion comprises an outer cover (not shown), the outer cover is provided with a yaw motor, a pitch motor and a clamping motor within. An output shaft end of the yaw motor is connected with an yaw motor active dial, an output shaft end of the pitch motor is connected with a pitch motor active dial, and an output shaft end of the clamping motor is connected with a clamping motor active dial; the yaw motor active dial is connected with a yaw motor passive dial, the pitch motor active dial is connected with a pitch motor passive dial, and the clamping motor active dial is connected with a clamping motor passive dial. The yaw motor passive dial, the pitch motor passive dial and the clamping motor passive dial are respectively connected with a wire winding apparatus.

Optionally, one end of the thin linking rod is fix arranged within the power portion and the other end of the thin linking rod extends out the power portion. Wherein, the thin linking rod is a hollow structure, and several fixing slots are provided within the hollow structure. The wire winding apparatus are fix arranged within the thin linking rod through the fixing slots.

Optionally, the wire winding apparatus are fix arranged within the thin linking rod through the fixing slots. The operative end portion connects with the thin linking rod at one end that is away from the power portion, and the operative end portion also connects with the wire winding apparatus.

Optionally, the side wall of the third linking rod is provided with a clutch button; the clutch button is arranged directly below the clamping unit and is wire connected with operative system.

Optionally, the third linking rod is further provided with a bracelet, and the bracelet is arranged directly below the clutch button. Wherein, the bracelet and the clutch button are arranged on a same bus bar.

Optionally, the bracelet which comprising a ring portion and connecting ends which are fix arranged at two ends of the ring portion. The connecting ends are fix mounted on the third linking rod. Wherein, the ring portion is made of elastic material and the connecting ends are made of hard material.

The multi DOF surgical instrument may provide yaw, pitch, clamping and axis rotation. When a surgeon holds the third linking rod, the surgeon can control the instrument to perform yaw, pitch and clamping motions through the operative end portion and the power potion. In addition, the turning of the surgeon's arm is reflected as an axis rotation of the operative end portion.

### BREIF DESCRIPTION OF THE DRAWINGS

In order that the invention may be well understood, an embodiment thereof, which is given by way of example only, will now be described with reference to the drawings in which:
Fig. 1 is a perspective view of a multi-DOF surgical instrument;
Fig. 2 is a perspective view of a hand brace of the surgical instrument;
Fig. 3 is a perspective view of a hand held portion of the surgical instrument;
Fig. 4 is a perspective view of a clamping unit of the surgical instrument;
Fig. 5 is an internal perspective view of one end of a power portion of the surgical instrument; and
Fig. 6 is an internal perspective view of the other end of the surgical instrument.

### DETAILED DECRIPTION

There follows a description of an embodiment of the invention. Those skilled in art can understand the advantages and other technical effects of the invention from the described embodiment. The terms of "first, second or third" used in connection with the embodiment are used only for reference for easier explanation and do not relate to the importance of the components or imply any other limitation.

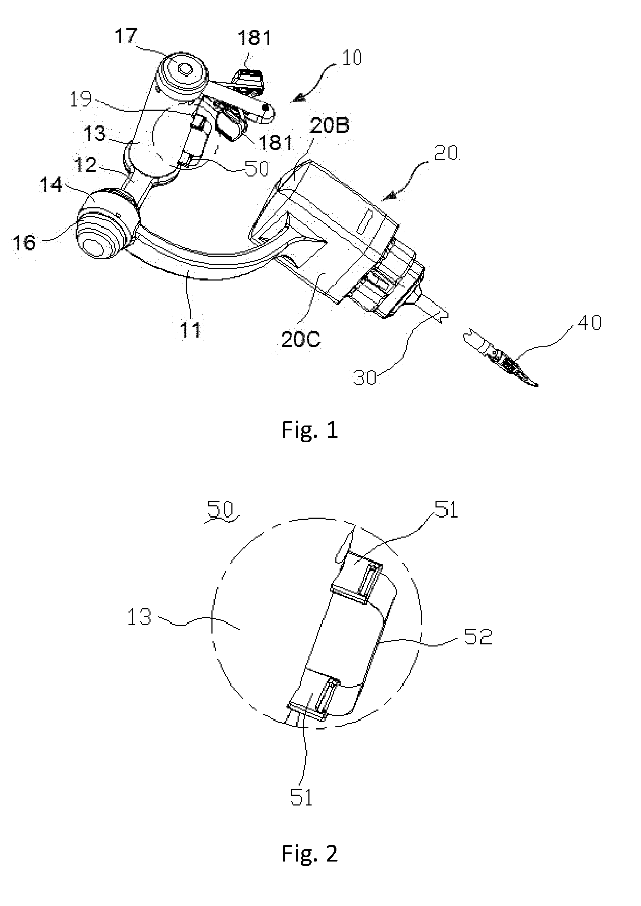

As shown in Fig. 1, a multi-degree of freedom (DOF) surgical instrument for minimally invasive surgery comprises a hand held portion 10, a power portion 20 which is fixedly connected with the hand held portion 10, a linking arm 30 which is fixedly connected with the power portion 20, an operative end portion 40 which is connected with the linking arm 30, and an operative system (not shown) for controlling the operation of the power portion 20. The operative, or control, system may comprise at least one of a chip, other suitable processor or processing circuitry.

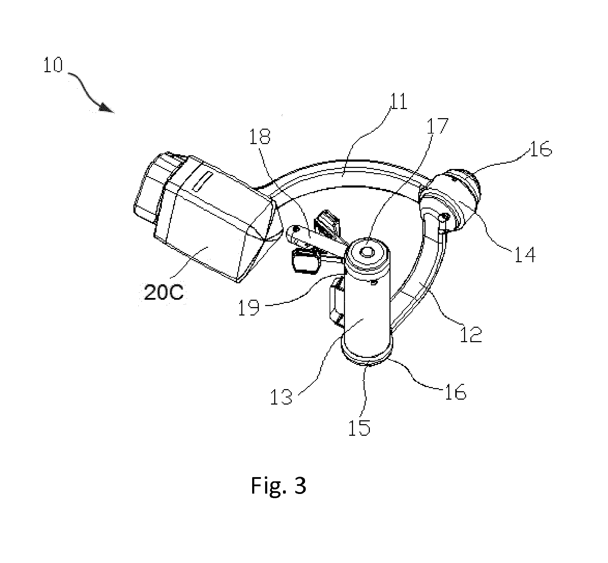

As shown in Fig. 3, the hand held portion 10 comprises a first linking member, or rod, 11. One end of the first linking rod 11 is fixedly connected with the power portion 20. The hand held portion 10 further comprises: a second linking member, or rod, 12 rotatably connected with the first linking rod 11 via a first joint 14; a third linking member, or rod, 13 rotatably connected with the second linking rod 12 via a second joint 15; a clamping unit 18 fixedly mounted on the top of the third linking rod 13 via a clamping cap 17; and respective encoders 16 fixedly mounted on the first joint 14 and the second joint 15.

The side wall of the third linking rod 13 is provided with a clutch button 19. The clutch button 19 is arranged directly below the clamping unit 18 and is connected with operative system by suitable wiring. When the hand of a surgeon reaches an operative movement limit, the clutch button 19 can be pressed to allow disengagement of the hand held portion 10 and the operative end portion 40 to allow adjustment of the position of the hand held portion relative to the operative end portion. Once the hand held portion 10 has been suitably adjusted to a new operative position, the clutch button 19 can be released to allow re-engagement and the surgeon can continue with the operation.

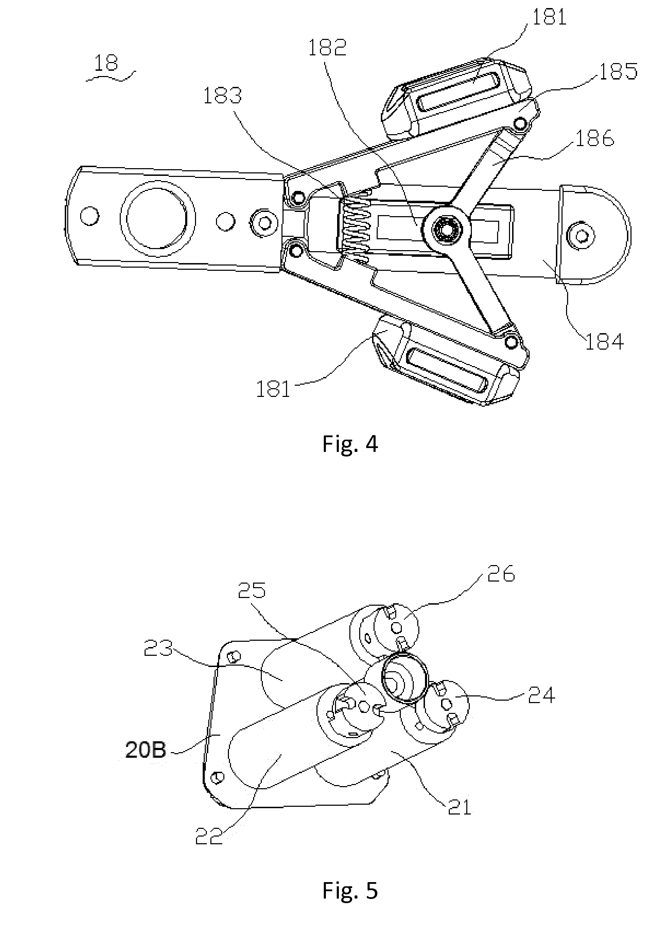

As shown in Fig. 4, the clamping unit 18 comprises a support such as a base panel, 184. The base panel 184 is provided with a pair of symmetrically opposing linkage mechanisms. Each of the linkage mechanisms comprises a mover lever 185 and a lower lever 186. One end of each mover lever 185 is rotatably connected with the base panel 184 and the other end of each mover lever 185 is hinged to one end of the respective lower lever 186. The other end of the lower lever 186 is rotatably connected with a protruding end of a potentiometer 182. The potentiometer 182 is fixedly mounted on the base panel 184. A spring 183 is provided between the mover levers 185 of the pair linkage mechanisms. Respective ends of the spring 183 abut against the two mover levers 185. The outer side walls of the mover levers 185 are provided with respective clamping pieces, pads or grips 181.

Referring to Figs 1 and 2, the third linking rod 13 is further provided with a hand brace 50 that is arranged directly below the clutch button 19. The hand brace 50 and the clutch button 19 are arranged in line in the same plane. The hand brace 50 comprises a cross member 52 and respective connecting ends 51 which are fixed to the two ends of the cross member 52. The connecting ends 51 are fixed to the third linking rod 13. The cross member 52 is made of an elastic material and the connecting ends 51 are made of a relatively harder, or stiffer, material. The cross member 52 is C shaped. A space is defined between the cross member 52 and the third linking rod 13 for receiving the three holding fingers of the surgeon. Since the cross member 52 is made of elastic material, it may deform when a surgeon's fingers are inserted into the space defined between the cross member 52 and the third linking rod 13 so that the space can adapt for use by different surgeons.

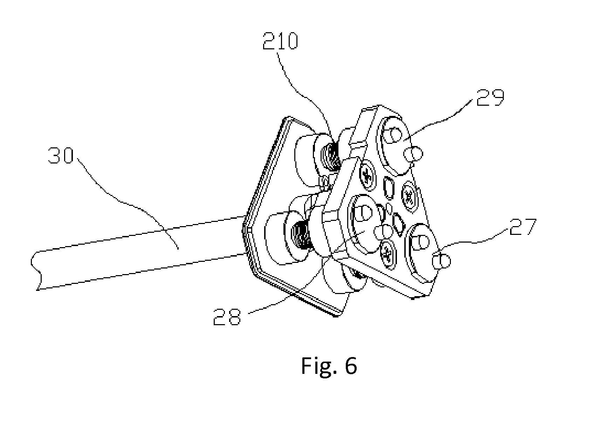

As shown in Figs 1 and 3, the power portion 20 comprises an outer cover 20C. Referring to Fig. 5, the outer cover 20C houses a yaw motor 21, a pitch motor 22 and a clamping motor 23. The motors 21, 22, 23 are mounted on a back plate 20B. An output shaft end of the yaw motor 21 is connected with a yaw motor output drive member 24, an output shaft end of the pitch motor 22 is connected with a pitch motor output drive member 25 and an output shaft end of the clamping motor 23 is connected with a clamping motor output drive member 26. Referring to Fig. 6, the yaw motor output drive member 24 is connected with a yaw motor drive receiving member 27, the pitch motor output drive member 25 is connected with a pitch motor drive receiving member 28 and the clamping motor output drive member 26 is connected with a clamping motor drive receiving member 29. The yaw motor drive receiving member 27, the pitch motor drive receiving member 28 and the clamping motor drive receiving member 29 are connected with wires of respective wire winding apparatus 210.

Referring to Fig. 6 ne end of the linking arm 30 is fixed within the power portion 20 while the other end extends outwardly from the power portion 20. The linking arm 30 is a hollow structure. Several fixing slots are provided within the linking arm 30. The wires of the winding apparatus 210 are fixed within the linking arm 30 via the fixing slots. The operative end portion 40 is connected with the end of the linking arm 30 that is remote from the power portion 20 (the leading, or free, end of the linking arm). The operative end portion 40 also connects with the wires of the wire winding apparatus 210.

The hand held portion 10 and the power portion 20 are both electrically connected with the operative system. Operation of the hand held portion 10 drives the respective encoders 16 on the joints 14, 15. The encoders 16 collect operative information relating to relative movement of the first linking rod and the second linking rod and the second linking rod and the third linking rod respectively and that information is fed back to the operative system. The power portion 20 drives the operative end portion 40 to complete yaw, pitch and clamping motions in response to signals received from the operative system that are based on the information received from the encoders 16.

When operating, a surgeon holds onto the third linking rod 13. The thumb and the index finger of the surgeon press and hold the clamping pieces 181 mounted on the two mover levers 185. The spring bias of the spring 183 holds the clamping piece 181 open automatically and the pressing force from the surgeon's fingers may cancel the force from the spring 183 to complete a closing action. During the clamping process the electrical resistance of the potentiometer 182 changes, causing the voltage of the potentiometer 182 to change. This change in voltage triggers an operative signal to cause the operative end portion 40 to perform a clamping action. The respective encoders 16 on the first joint 14 and the second joint 15 collect information from the second linking rod 12 and the third linking rod 13 and feed this back to the operative system. The power portion 20 controls the operative end portion 40 to perform corresponding yaw and pitch motions. In addition, the turning of the arm of the surgeon can be reflected as an axis rotation of the operative end portion 40.

Although the invention has been explained in relation to the described embodiment, it is to be understood that there are many possible modifications and variations that can be made without departing from the scope of the invention as hereinafter claimed.

## Claims

1. A multi-degree of freedom (DOF) surgical instrument for minimally invasive surgery comprising a hand held portion (10), a power portion (20) fixedly connected with the hand held portion (10), a linking arm (30) fixedly connected with the power portion (20), an operative end portion (40) connected with the linking arm (30), and an operative system for controlling the operation of the power portion (20), wherein
the hand held portion (10) comprises a first linking member(11) having a first end fixedly connected with the power portion (20), a second linking member (12) rotatably connected with the first linking member (11) via a first joint (14), a third linking member (13) rotatably connected with the second linking member (12) via a second joint (15), a clamping unit (18) fixed to the third linking member (13), and respective encoders (16) which are fixed to the first and second joints (14, 15),
the clamping unit (18) comprises a support (184) provided with a pair of opposed linkage mechanisms and a spring (183), each of the linkage mechanisms comprising a mover lever (185) and a lower lever (186), a first end of each mover lever 185 being rotatably connected with the support (184) the second end of each mover lever (185) being hinge connected with a first end of the respective lower lever (186), the second end of each lower lever (186) being rotatably connected with a potentiometer (182) mounted on the support (184), and the spring (183) being provided between the mover levers (185)and having respective ends acting against the two mover levers (185), and
during operation of the hand held portion (10) the respective encoders (16) (output signals indicative of relative movement of the first and second linking members (11, 12) and of the second and third linking members (12, 13) and the operative system provides signals to the power portion (20) to drive the operative end portion (40) to complete yaw, pitch and clamping motions based on the signals output by the encoders (16).

2. A surgical instrument as claimed in claim 1 wherein
the power portion (20) comprises a yaw motor (21), a pitch motor (22) and a clamping motor (23),
an output shaft end of the yaw motor (21) is provided with a yaw motor output drive member (24), an output shaft end of the pitch motor (22) is provided with a pitch motor output drive member (25), and an output shaft end of the clamping motor (23) is connected with a clamping motor output drive member (26),
the yaw motor output drive member (24) is connected with a yaw motor drive receiving member (27), the pitch motor output drive member (25) is connected with a pitch motor drive receiving member (28), and the clamping motor output drive member (26) is connected with a clamping motor drive receiving member (29), and
the yaw motor drive receiving member (27), the pitch motor drive receiving member (28) and the clamping motor drive receiving member (29) are connected with wire winding apparatus (210).

3. A surgical instrument as claimed in claim 2, wherein a first end of the linking arm (30) is fixed within the power portion (20), a second end of the linking arm (30) is disposed remote from the power portion (20), the linking arm (30) is a hollow structure provided with a plurality of fixing slots and wires of the winding apparatus (210) are fix within the linking arm (30) via the fixing slots.

4. A surgical instrument as claimed in claim 3, wherein the operative end portion (40) is connected with the second end of the linking arm (30) and the wires of the wire winding apparatus (210).

5. A surgical as claimed in any one of the preceding claims, further comprising a clutch button (19) provided on the third linking member (13), said clutch button (19) being operable to disengage the hand held portion (10) and operative end portion (40) to permit repositioning of the hand held portion relative to the operative end portion.

6. A surgical instrument as claimed in claim 5, wherein said clutch button (19) is disposed below the clamping unit (18) and is electrically connected with the operative system.

7. A surgical instrument as claimed in any one of the preceding claims, further comprising a hand brace (50) provided on the third linking member (13), said hand brace (50) comprising a cross member (52) and respective connecting ends (51) provided at the two ends of the cross member (52), said connecting ends (51) being fixedly mounted on the third linking member (13) and the cross member (52) being made of an elastic material that is deformable in response to insertion of a surgeon's fingers between said cross member and third linking member.

8. A surgical instrument as claimed in claim 7 when dependent on claim 5 or 6, wherein the hand brace (50) is arranged directly below and in the same plane as the clutch button (19).
